**Europäisches Patentamt**

**European Patent Office**

**Office européen des brevets**

(11) Veröffentlichungsnummer: **0 151 785**
**B1**

# (12) EUROPÄISCHE PATENTSCHRIFT

(45) Veröffentlichungstag der Patentschrift:
18.03.87

(21) Anmeldenummer: 84116068.2

(22) Anmeldetag: 21.12.84

(51) Int. Cl.⁴: **C 07 C 11/167,** C 07 C 11/08, C 07 C 7/10, C 07 C 7/04

(54) **Verfahren zur Kapazitätssteigerung einer Extraktionsanlage zur Gewinnung von Butadien-(1,3) aus C4-Kohlenwasserstoffgemischen unter gleichzeitiger Gewinnung von Buten-(2).**

(30) Priorität: 15.02.84 DE 3405338

(43) Veröffentlichungstag der Anmeldung:
21.08.85 Patentblatt 85/34

(45) Bekanntmachung des Hinweises auf die Patenterteilung:
18.03.87 Patentblatt 87/12

(84) Benannte Vertragsstaaten:
AT BE DE FR GB IT NL

(56) Entgegenhaltungen:
US-A-2 925 452

(73) Patentinhaber: BUNAWERKE HÜLS GMBH,
Postfach 1320, D-4370 Marl 1 (DE)

(72) Erfinder: Donike, Wilhelm, Dr., Uerdinger Strasse 3, D-4370 Marl (DE)
Erfinder: Ulrich, Helmut, Krampenfeld 48, D-4270 Dorsten (DE)

## Beschreibung

Die beiden 2-Butene, cis-Buten-(2) und trans-Buten-(2), haben bislang keine große technische Bedeutung erlangt. Sie wurden im Gemisch mit Buten-1 hauptsächlich zur Herstellung von Maleinsäureanhydrid und sec.-Butanol verwendet. Erst in jüngerer Zeit wurden sie für einige Einsatzgebiete als Rohstoff interessant, z.B. für die Herstellung von Epoxiden, Methylethylketon und als Copolymeres. Voraussetzung für diese Verwendung ist jedoch ein guter Reinheitsgrad.

Die 2-Butene stehen im Gemisch mit anderen $C_4$-Kohlenwasserstoffen in großen Mengen zur Verfügung. Sie sind zwar in den Raffinerieabgasen und den $C_4$-Kohlenwasserstoffgemischen enthalten, wie sie beim Betrieb von Steamcrackern zur Ethylenerzeugung anfallen; die Gewinnung der Einzelkomponenten aus diesen $C_4$-Kohlenwasserstoffgemischen ist jedoch durch die nahe beieinander liegenden Siedepunkte und Azeotropbildung der $C_4$-Kohlenwasserstoffe relativ aufwendig.

Ein typischer $C_4$-Schnitt aus einem Steamcracker hat folgende Zusammensetzung:

$C_3$-KW 0,03
i-Butan 2,48
n-Butan 6,14
Buten-(1) 11,70
i-Buten 28,78
trans-Buten-(2) 4,39
cis-Buten-(2) 3,82
Butadien-(1,3) 41,54
Butadien-(1,2) 0,22
Butin-(1) 0,13
Butenin 0.77

Die Aufarbeitung erfolgt normalerweise in mehreren Stufen, wobei einige Varianten möglich sind:

1. Abtrennung der Leichtsieder durch Destillation.

2. Extraktion von Butadien-(1,3) mit Hilfe eines selektiven Lösemittels bei gleichzeitiger Abtrennung von Butenin, Butin-(1) und Butadien-(1,2).

3. Extraktion von i-Buten mit Schwefelsäure oder Umsetzung des i-Butens zu MTB (Methyl-tert.-butylether).

4. Trennung der Butane von den Butenen durch Extraktivdestillation mit Hilfe eines selektiven Lösemittels.

5. Abtrennung von Buten-(1) von Buten-(2) durch Destillation.

Aus wirtschaftlichen Gründen werden normalerweise nur die ersten drei Verfahrensschritte ausgeführt, d. h., es wird nur Butadien und i-Buten isoliert bzw. aus dem Gemisch entfernt. Das verbleibende Gemisch der Butene und Butane wird nicht weiter aufgetrennt, sondern als solches einer weiteren Verwendung zugeführt.

Bei der Extraktion von Butadien aus den sogenannten $C_4$-Schnitten werden verschiedene Verfahren angewandt. Durchgesetzt haben sich Extraktivdestillationsverfahren, wobei als Extraktionsmittel polare, organische Lösemittel eingesetzt werden. Die wichtigsten Extraktionsmittel dieser Art sind N-Methylpyrrolidon (NMP), Dimethylformamid (DMF) und Dimethylacetamid (DMA). Die polaren, organischen Lösemittel bewirken eine Veränderung der relativen Flüchtigkeit der $C_4$-Kohlenwasserstoffe, so daß eine Abtrennung von reinem Butadien-(1,3) möglich wird. Die "Schlüsselkomponenten", d. h., die auf Grund ihrer relativen Flüchtigkeit dem Butadien-(1,3) am nächsten liegenden Komponenten sind cis-Buten-(2), trans-Buten-(2) und Butin-1.

Diese sind naturgemäß besonders schwer abtrennbar und bestimmen die Kapazität der entsprechenden Extraktionsanlage.

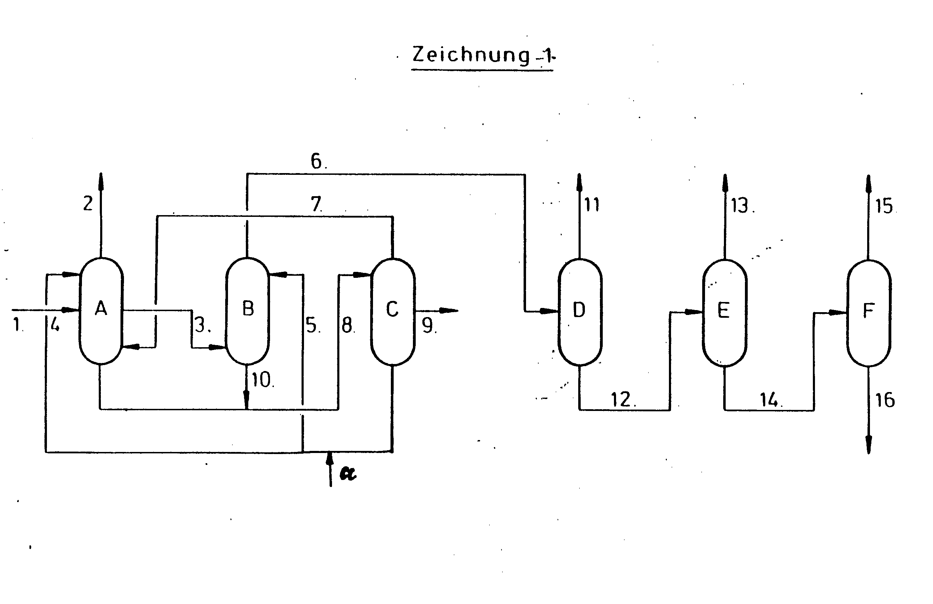

Zur Erläuterung des Sachverhaltes dient die Zeichnung 1.

Als übliche Verfahrensweise wird der Extraktionsstufe A über die Leitung 1 butadienhaltiger $C_4$-Schnitt zugeführt, während über die Leitung 4 Lösemittel eingespeist wird. Die Butene und Butane fallen als sogenanntes Raffinat am Kopf der Extraktionskolonne A an und werden über die Leitung 2 abgezogen. Im Raffinat ist die Hauptmenge der 2-Butene enthalten.

Das im Sumpf der Extraktionskolonne A anfallende Gemisch (gelöstes Butadien-(1,3), $C_4$-Acetylene und Lösemittel) wird über die Leitung 8 in die Destillationskolonne C verpumpt, die normalerweise bei einem niedrigeren Druck als die Extraktionskolonne A betrieben wird. In der Destillationskolonne C wird das $C_4$-Gemisch aus dem Lösemittel abgestrippt, welches über die Leitungen 4 und 5 zu den Extraktionskolonnen A und B zum erneuten Einsatz als Lösemittel verpumpt wird. Über die Leitung a können verfahrensbedingte Lösungsmittelverluste ausgeglichen werden. Die $C_4$-Acetylene verlassen über den Seitenabzug 9 das System. Das am Kopf der Kolonne C anfallende Gas (vorwiegend Butadien-(1,3)) wird über Leitung 7 zur Kolonne A als Rückgas zurückgeführt.

Die zweite Extraktionskolonne B wird vom Seitenabzug der ersten Extraktionskolonne A über Leitung 3 gespeist und am Kopf über Leitung 5 mit Lösemittel beaufschlagt. In der Kolonne B werden $C_4$-Acetylene zurückgewaschen. Am Kopf der Kolonne B fällt Rohbutadien an, welches noch geringe Reste (0,3 %) cis-Buten-(2), $C_5$-Kohlenwasserstoffe und Butadien-(1,2) enthält. Über die Leitung 6 verläßt das Rohbutadien die Extraktion und wird der zweiten Destillationskolonne D zugeführt. In der Kolonne D wird destillativ im Sumpf das restliche cis-Buten-2, Butadien-(1,2) und $C_5$-Kohlenwasserstoffe abgetrennt. Dieses Gemisch verläßt die Kolonne D über Leitung 12. Am Kopf der Kolonne D fällt Reinbutadien mit weniger als 0,3 % cis-Buten-(2) an und wird über Leitung 11 der weiteren Verwendung zugeführt.

Die Kapazität einer Extraktionsanlage ist begrenzt durch die zuführbaren Mengen an Lösemittel und C$_4$-Kohlenwasserstoffen. Werden gewisse Grenzen, die von der Bauart der Kolonnen abhängig sind, überschritten, werden Kolonnenteile geflutet und ein Weiterbetreiben der Anlage ist unmöglich.

Hieraus ergab sich die Aufgabe, ein Verfahren zu finden, das es ermöglicht, die maximal durchsetzbare Menge am C$_4$-Kohlenwasserstoffgemisch zu erhöhen und gleichzeitig die 2-Butene kostengünstig zu gewinnen.

Diese Aufgabe wird erfindungsgemäß entsprechend den Angaben der Patentansprüche gelöst.

Die am Kopf der zweiten Extraktionskolonne B über Leitung 6 abgezogene Menge wird soweit erhöht, daß bis zu 3 Gew.-% cis-Buten-(2) im Rohbutadien, das einen Reinheitsgehalt von weniger als 99 % aufweist, enthalten ist. Das mit Butadien beladene Extraktionsmittel wird über 10 abgezogen. Im allgemeinen führt man es über 8 in die Destillationskolonne C. Man kann es auch in die Extraktionskolonne A zurückführen. Gleichzeitig wird aus dem Sumpfablauf der zweiten Destillationskolonne D der Strom über Leitung 12 soweit erhöht, daß man am Kopf der Kolonne D ein Reinbutadien mit einer Reinheit von über 99,5 % und mit weniger als 0,5 %, vorzugsweise weniger als 0,3 % cis-Buten-(2) erhält.

Die maximal durchsetzbare Menge am C$_4$-Kohlenwasserstoffgemisch erhöht sich durch diese Maßnahme überraschenderweise um bis zu 10 %. Diese Kapazitätssteigerung erbringt erhebliche wirtschaftliche Vorteile.

Das im Sumpf der zweiten Destillationskolonne D anfallende Produkt wird über Leitung 12 einer weiteren, dritten Destillationskolonne E zugeführt, in der Reinst-butadien-(1,3) über Kopf abgetrennt wird. In einer Destillationskolonne mit in praktischen Böden wird überraschenderweise ein sehr reines, für Polymerisationszwecke geeignetes Butadien-(1,3) gewonnen. Der Sumpf der Kolonne E wird über Leitung 14 einer vierten Destillationskolonne F zugeführt, an deren Kopf ein 2-Buten-Gemisch mit über 97 %iger Reinheit anfällt. Dieses ist als solches oder nach einer Hydrierstufe zur Entfernung von C$_4$-Acetylenen als reines 2-Buten einsetzbar. Aus dem Sumpf der Destillationskolonne F trennt man die höhersiedenden Kohlenwasserstoffe ab.

Zur weiteren Erläuterung dienen die nachfolgenden Beispiele.

**Vergleichsbeispiel A**

Eine Extraktionsanlage zur Gewinnung von Butadien-(1,3) ist mit maximal 29,0 t/Stunde (stuto) C$_4$-Schnitt belastbar, dessen Butadiengehalt 43,0 Gew.-% beträgt.

Am Kopf der Kolonne B fallen 12,22 stuto Rohbutadien an, das einen Reinheitsgehalt von 99,0 Gew.-% aufweist. Die Verunreinigungen bestehen aus 0,5 Gew.-% cis-Buten-(2) und 0,5 Gew.-% Hochsiedern.

In der Kolonne D fallen am Kopf 12,00 stuto Reinbutadien mit 0,3 Gew.-% cis-Buten-(2) an, während im Sumpf der Kolonne D 0,22 stuto einer Fraktion anfallen, die aus 50 Gew.-% Hochsiedern, 25 Gew.-% Butadien-(1,3), 3 Gew.-% Butin-(1) und 22 Gew.-% cis-Buten-(2) besteht. Diese Fraktion wird üblicherweise einem Heizgassystem zugeführt.

**Beispiel 1**

Die nach Vergleichsbeispiel A betriebene Extraktionsanlage ist mit 30,50 stuto C$_4$-Schnitt belastbar, wenn am Kopf der Kolonne B 12,96 stuto Rohbutadien mit einer Reinheit von 98,1 Gew.-% Butadien-(1,3) abgezogen werden. Die Verunreinigungen bestehen hauptsächlich aus 1,5 Gew.-% cis-Buten-(2) und 0,4 Gew.-% Hochsiedern.

In der Kolonne D fallen 12,51 stuto Reinbutadien an, das eine Reinheit von 99,6 Gew.-% und einen cis-Buten-(2)-Gehalt von 0,4 % aufweist.

Um diese Reinheit einzuhalten, werden im Sumpf der Kolonne D 0,45 stuto einer C$_4$-KW-Fraktion abgezogen, die folgende durchschnittliche Zusammensetzung aufweist:

Butadien-(1,3) 50,00 Gew.-%
cis-Buten-(2) 30,00 Gew.-%
Hochsieder 19,44 Gew.-%
trans-Buten-(2) 0,06 Gew.-%
Butin-(1) 0,50 Gew.-%

Diese Fraktion wird in der Kolonne E weiterverarbeitet, wobei im Kopf der Kolonne E überraschenderweise ein für Polymerisationszwecke brauchbares Butadien-(1,3) (0,22 stuto) anfällt, welches eine Reinheit von 99,7 Gew.-% aufweist. Es enthält 0,3 Gew.-% cis-Buten-(2) und keine störenden Bestandteile wie z. B. Butin-1 oder Hochsieder.

Der Sumpf der Kolonne E wird der Kolonne F zugeführt, wo als Kopfprodukt 0,135 stuto cis-Buten-(2) mit einer Reinheit von 95 Gew.-% anfällt. Die Verunreinigungen bestehen aus 3,0 Gew.-% Butadien-(1,3), 0,5 Gew.-% Hochsiedern und 1,5 Gew.-% Butin-1.

Für eine Weiterverwendung des cis-Buten-(2) stört u. U. der Gehalt von 1,5 Gew.-% Butin-(1), welches jedoch auf einfache Weise durch eine selektive Hydrierung zu nichtstörenden Butenen umgesetzt werden kann.

**Patentansprüche**

1. Verfahren zur Kapazitätssteigerung einer Extraktionsanlage zur Gewinnung von Butadien-1,3 aus C$_4$-KW-Gemischen und zur Gewinnung von Buten-2 durch Extraktion eines

butadienhaltigen $C_4$-Schnittes in einer ersten Extraktionsstufe A und einer zweiten Extraktionsstufe B mit einer dazwischengeschalteten Destillationskolonne C und einer nachgeschalteten Destillationskolonne D,

dadurch gekennzeichnet,

daß man einen Teil des Buten-2 mit dem Rohbutadien aus der zweiten Extraktionsstufe B abzieht und durch mehrere nachgeschaltete Destillationsstufen vom Butadien-1,3 und höhersiedenden Kohlenwasserstoffen trennt, wobei man die am Kopf der zweiten Extraktionsstufe B abgezogene Menge so weit erhöht, daß sie bis zu 3 Gewichtsprozent cis-Buten-2 im Rohbutadien, das einen Reinheitsgehalt von weniger als 99% aufweist, enthält, gleichzeitig die Sumpfabnehme der nachgeschalteten zweiten Destillationsstufe D so weit erhöht, daß man am Kopf dieser Destillationskolonne ein Reinbutadien mit einer Reinheit von über 99,5% und mit weniger als 0,5% cis-Buten-2 erhält, den Sumpf dieser Kolonne in eine dritte Destillationsstufe E gibt, in der man als Kopfprodukt das restliche Reinbutadien erhält, und den Sumpf der dritten Destillationsstufe E in eine vierte Destillationsstufe F führt, in der man als Kopfprodukt hochprozentiges Buten-2 gewinnt.

2. Verfahren nach Anspruch 1,

dadurch gekennzeichnet,

daß man die Sumpfabnehme der zweiten Destillationsstufe D so weit erhöht, daß man am Kopf dieser Kolonne ein Reinbutadien mit weniger als 0,3 % cis-Buten-2 erhält.

## Claims

1. A process for increasing the capacity of an extraction plant for the recovery of 1,3-butadiene from $C_4$-hydrocarbon mixtures and for the recovery of 2-butene by extraction of a butadiene-containing $C_4$-cut in a first extraction stage A and a second extraction stage B with an intervening distillation column C and a subsequent distillation column D, characterised in that a part of the 2-butene is withdrawn with the crude butadiene from the second extraction stage B and is separated from 1,3-butadiene and higher boiling hydrocarbons by means of a plurality of subsequent distillation stages, the amount withdrawn at the head of the second extraction stage B being increased to such an extent that it contains up to 3% by weight of cis-2-butene in the crude butadiene which has a purity level of less than 99%, simultaneously the amount withdrawn from the sump of the subsequent second distillation column D being increased to such an extent that a pure butadiene with a purity of more than 99. 5% and a cis-2-butene content of less than 0.5% is obtained at the head of this distillation column, the bottoms from this column being fed to a third distillation stage E in which the remainder of the pure butadiene is obtained as overhead product, and the bottoms product of the third distillation stage E being passed to a fourth distillation stage F in which high-purity 2-butene is recovered as overhead product.

2. A process according to claim 1, characterised in that the amount withdrawn from the sump of the second distillation stage D is increased to such an extent that a pure butadiene with less than 0.3 % cis-2-butene is obtained at the head of this column.

## Revendications

1. Procédé pour l'augmentation de la capacité d'une installation d'extraction pour l'obtention de butadiène-1,3 en partant de mélanges d'hydrocarbures en $C_4$ et pour l'obtention de butène-2 par extraction d'une fraction en $C_4$ contenant du butadiène en une première étape d'extraction A et en une deuxième étape d'extraction B, avec une colonne de distillation C interposée entre celles-ci et une colonne de distillation D branchée à la suite, caractérisé par le fait que l'on retire de la deuxième étape d'extraction B une partie du butène-2 avec le butadiène brut et qu'on le sépare du butadiène-1,3 et des hydrocarbures à point d'ébullition plus élevé par plusieurs étapes de distilation faisant suite, en augmentant la quantité retirée à la tête de la deuxième étape d'extraction B dans une mesure telle qu'elle contient jusqu'à 3 % en poids de cis-butène-2 dans le butadiène brut, qui présente une pureté de moins de 99 %, qu'en même temps on augmente le retrait de queues de la deuxième étape de distillation D branchée à la suite, dans une mesure telle que l'on obtient à la tête de cette colonne de distillation un butadiène pur d'une pureté de plus de 99,5 % et contenant moins de 0,5 % de cis-butène-2, que l'on amène les queues de cette colonne à une troisième étape de distillation E, dans laquelle on obtient comme produit de tête le butadiène pur résiduel, et que l'on amène les queues de la troisième étape de distillation E à une quatrième étape de distillation F, dans laquelle on obtient comme produit de tête du butène-2 à pourcentage élevé.

2. Procédé selon la revendication 1, caractérisé par le fait que l'on augmente le retrait de queues de la deuxième étape de distillation D dans une mesure telle que l'on obtient à la tête de cette colonne un butadiène pur contenant moins de 0,3 % de cis-butène-2.

Zeichnung 1